(19) **Europäisches Patentamt European Patent Office Office européen des brevets**

(11) **EP 4 407 628 B1**

(12) **EUROPEAN PATENT SPECIFICATION**

(45) Date of publication and mention of the grant of the patent:
**04.06.2025 Bulletin 2025/23**

(51) International Patent Classification (IPC):
**G16H 20/30** (2018.01)    **G16H 40/67** (2018.01)
**G16H 50/20** (2018.01)

(21) Application number: **23153803.4**

(52) Cooperative Patent Classification (CPC):
**G16H 50/20; G16H 20/30;** G16H 40/67

(22) Date of filing: **27.01.2023**

(54) **A SYSTEM AND METHOD FOR IDENTIFYING, ANALYSING AND COUNTING REPETITIONS OF PHYSICAL EXERCISES BY AN INDIVIDUAL, IN PARTICULAR FOR USE IN REHABILITATION PROGRAMS**

SYSTEM UND VERFAHREN ZUR IDENTIFIZIERUNG, ANALYSE UND ZÄHLUNG VON WIEDERHOLUNGEN KÖRPERLICHER ÜBUNGEN DURCH EINE PERSON, INSBESONDERE ZUR VERWENDUNG IN REHABILITATIONSPROGRAMMEN

SYSTÈME ET PROCÉDÉ D'IDENTIFICATION, D'ANALYSE ET DE COMPTAGE DE RÉPÉTITIONS D'EXERCICES PHYSIQUES PAR UN INDIVIDU, EN PARTICULIER POUR UNE UTILISATION DANS DES PROGRAMMES DE RÉÉDUCATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**31.07.2024 Bulletin 2024/31**

(73) Proprietor: **ARC Intellicare Ltd**
**Lisburn, BT28 2EX (GB)**

(72) Inventors:
• **LAI, Stefano**
  **43123 Parma (IT)**
• **MANTOAN, Alice**
  **43123 Parma (IT)**

• **SERNISSI, Francesca**
  **43123 Parma (IT)**
• **ASCARI, Luca**
  **43123 Parma (IT)**
• **SYMONS, Frederick**
  **43123 Parma (IT)**

(74) Representative: **Dondi, Silvia**
**Bugnion S.p.A.**
**Largo Michele Novaro, 1/A**
**43121 Parma (IT)**

(56) References cited:
**WO-A1-2020/205276    US-A1- 2022 080 262**

**Description**

The present invention relates to a system and method for identifying, analysing, and counting repetitions of physical exercises by an individual, in particular for use in rehabilitation programs.

The invention operates in the physical rehabilitation field, with the scope of helping patients by supporting them in their rehabilitation programs provided by a physician.

There are already known in the art systems and methods for monitoring patients in need of physiotherapy.

For example, document EP 3627514 A1 describes a system comprising three-axes accelerometers and three-axes gyroscopes as wireless motion sensors, attached around the injured joint, and detecting, monitoring, and reporting the quality of the exercises patients are doing both to the physiotherapist and to the patient.

From document AU 2020101986 A4 it is known a physical activity monitoring device receiving a workout regimen including a plurality of exercises. The device outputs information regarding user's progress toward completion of the selected exercise, based on physical activity attributes.

The device is based on a support vector machine for identifying exercises, and it counts repetition in real time by peak-counting.

Another method for exercise recognition is proposed in WO 2020/205276 A1. This method includes identifying repetitions of an exercise being performed by the user based on the motion data received from the at least one motion sensor. The method further includes calculating a performance score for the user based on the motion data received from the at least one motion sensor, wherein the performance score is calculated using a neural network trained using feedback provided by one or more expert reviewers based on review of video of one or more training users performing the exercise.

US 2022/080262 A1 discloses a method and an apparatus to generate motion data and process them with the aim of providing new personalized performance metrics learned through machine learning techniques.

There is felt the need of solutions that work equally well on both healthy and impaired subjects, and that are applicable even to complex exercises.

In this context, the technical task at the basis of the present invention is to propose a system and method for identifying, analysing, and counting repetitions of physical exercises by an individual, which overcome the drawbacks of the prior art cited above.

In particular, it is an object of the present invention to propose a system and method for identifying, analysing, and counting repetitions of physical exercises by an individual, which allow to perform physical rehabilitation programs without the physical presence of a physician.

More specifically, another object of the present invention is to propose a system and method for identifying, analysing, and counting repetitions of physical exercises by an individual, which allow the rehabilitation of patients with motor-movement disorders.

Another object of the present invention is to propose a system and method for identifying, analysing, and counting repetitions of physical exercises by an individual, that is applicable also to complex exercises, composed of a sequence of movements.

Another object of the present invention is to propose a system and method for identifying, analysing, and counting repetitions of physical exercises by an individual, that is more flexible than prior art solutions, in that it can be scaled by adding new exercises based on specific needs.

The stated technical task and specified objects are substantially achieved by a system for identifying, analysing, and counting repetitions of physical exercises by an individual, in particular for use in rehabilitation programs, according to independent claim 1 and to dependent claims 2 to 11.

The stated technical task and specified objects are substantially achieved by a computer-implemented method for identifying, analysing, and counting repetitions of physical exercises by an individual, in particular for use in rehabilitation programs, according to independent claim 12 and to dependent claims 13-14.

Additional features and advantages of the present invention will become more apparent from the non-limiting, description of a preferred, but nonexclusive embodiment of a system and method for identifying, analysing, and counting repetitions of physical exercises by an individual, as illustrated in the appended drawings, in which:

- figures 1a, 1b schematically illustrate a system for identifying, analysing, and counting repetitions of physical exercises by an individual, in particular for use in rehabilitation programs, according to two different embodiments of the present invention;

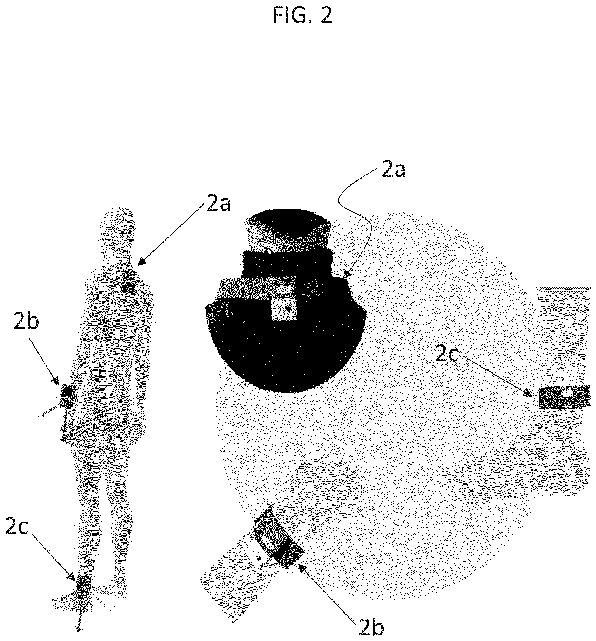

- figure 2 illustrates some possible body positions for inertial sensors of the system of figure 1;

- figure 3 illustrates the block diagram of the residual neural network of the system of figure 1;

- figure 4 illustrates the dataflow of the first neural network of the system of figure 1;

- figure 5 is relative to a first example of exercise to be recognized in the system of figure 1;

- figure 6 is relative to a second example of exercise to be recognized in the system of figure 1;

- figure 7 is a table summarizing exercise complexity definition;

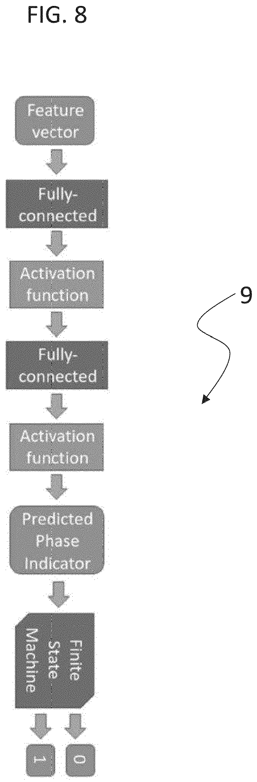

- figure 8 illustrates the dataflow of the third neural network of the system of figure 1;

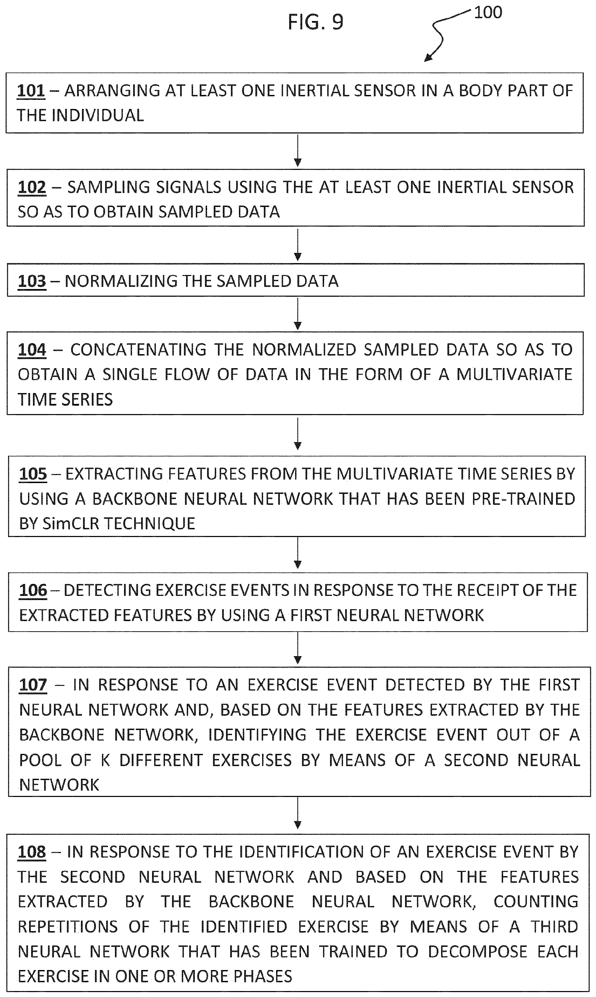

- figure 9 illustrates a flowchart of a method for counting repetitions of physical exercises by an individual, according to the present invention.

With reference to the figures, number 1 indicates a system comprising at least one inertial sensor 2 that is wearable by an individual, for example a patient of a rehabilitation program.

Each inertial sensor 2 is configured to sample corresponding signals which are representative of biometric and/or medical parameters of the individual so as to obtain sampled data.

In particular, the inertial sensor 2 is configured to sample corresponding inertial data, i.e., accelerations and angular velocities, which are representative of the individual movement of the individual so as to obtain sampled data.

According to an aspect of the invention, the inertial sensor 2 is a 6-axis inertial sensor comprising an accelerometer and a gyroscope, which are referred to as "sensing elements" in this context.

In particular, each inertial sensor 2 is configured to sample signals at a frequency of at least 30 Hz, and to stream the acquired signal by BLE (acronym for "Bluetooth Low Energy") technology.

Inertial sensor 2 used in the system 1 are already known in the market and will not be further detailed.

In the preferred embodiment of the invention, there are three inertial sensors 2 so arranged:

- a first inertial sensor 2a applicable to the back neck of the individual;
- a second and a third inertial sensors 2b, respectively applicable to the right wrist and left wrist of the individual.

This configuration of inertial sensors 2a, 2b is used for monitoring the neck and the upper limbs.

In another embodiment of the invention, there are three inertial sensors 2 so arranged:

- a first inertial sensor 2a applicable to the back neck of the individual;
- a second and a third inertial sensors 2c, respectively applicable to the right ankle and left ankle of the individual.

This configuration of inertial sensors 2a, 2c is used for monitoring the neck and the lower limbs.

In another embodiment of the invention, there are five inertial sensors 2 so arranged:

- a first inertial sensor 2a applicable to the back neck of the individual;
- a second and a third inertial sensors 2b, respectively applicable to the right wrist and left wrist of the individual;
- a fourth and fifth inertial sensors 2c, respectively applicable to the right ankle and left ankle of the individual.

This configuration of inertial sensors 2a, 2b, 2c is used for monitoring the neck, the upper limbs, and the lower limbs.

There are envisaged also other embodiments using a different number of inertial sensors 2, scattered in different body parts of the individual.

The sampled data coming from the inertial sensors 2 are channelized in a plurality of channels 3 and provided as input to a normalization unit 4.

The incoming sampled data arrives at the normalization unit 4 in raw batches of potentially unequal length.

In particular, in the embodiments with three inertial sensors 2, the system 1 comprises eighteen channels 3, where eighteen is given by three inertial sensors multiplied by two sensing elements multiplied by three axes.

In the embodiment with five inertial sensors 2, the system 1 comprises thirty channels 3, where thirty is given by five inertial sensors multiplied by two sensing elements multiplied by three axes.

In the embodiment with one inertial sensor 2, the system 1 comprises six channels 3, where six is given by one inertial sensor multiplied by two sensing elements multiplied by three axes.

In particular, each channel 3 is configured to transmit the sampled data acquired from the corresponding inertial sensor 2 to the normalization unit 4.

In each channel 3 the sampled data are transmitted in a chronological order, that is the order of acquisition by the corresponding inertial sensor 2.

The normalization unit 4 is configured to apply a normalization formula to the sampled data of each channel 3.

In particular, each channel 3 of sampled data has a first constant value subtracted and is then divided by a second constant value.

For example, the first constant value is the mean of a previously observed dataset of that channel 3, and the second constant value is the standard deviation of a previously observed dataset of that channel 3.

Preferably, the normalization unit 4 comprises a buffer to store normalized data. This allows more flexibility with data arriving in batches of irregular length.

The normalized data exiting the normalization unit 4 are sent as input to a fusion unit 5 that in response is configured to provide as output a single flow data obtained at least as a concatenation of the data coming from the normalization unit 4.

In particular, when the buffer of the normalization unit 4 is full, then it starts the transmission of the stored normalized data from the buffer to the fusion unit 5.

The fusion unit 5 serves for converting the normalized data to an internal format suited for further processing.

The fusion unit 5 allows to use a variable number of inertial sensors 2, allowing the system 1 to automatically attribute importance to the sensors relevant for each exercise.

According to one embodiment, the fusion unit 5 is configured to concatenate the data coming from the normalization unit 4 and then to further normalize them.

According to another embodiment, the fusion unit 5 is configured to concatenate the data coming from the normalization unit 4 and then to apply a transform function so as to eliminate unnecessary data, for example uninformative dimensions.

In particular, transform functions for environmental noise reduction may be applied, which are already known in the art.

In particular, in the embodiments with three inertial sensors 2, the fusion unit 5 receives as input an 18-dimensional signal $[\mathbf{a}(t, j), \mathbf{g}(t, j)]$ where the index $j = 1, ..., N$ denotes the sensor index, $t$ is the time measurement and both $\mathbf{a}$ and $\mathbf{g}$ are vectors of length 9 = 3 * 3 (3 mount-points * 3 dimensions that are x, y, z).

After the concatenation operation an 18 * N dimensional signal is obtained:

$$[[\mathbf{a}(t, 1), \mathbf{g}(t, 1)], [\mathbf{a}(t, 2), \mathbf{g}(t, 2)], ..., [\mathbf{a}(t, N), \mathbf{g}(t, N)]]$$

In the embodiments with more than three inertial sensors 2, the same concatenation operation is performed so as to obtain a 6 * i * N dimensional signal where i > 3 is the number of inertial sensors 2 used, and then apply a transform, for example a dimensionality reduction operation.

For example, the following dimension-reduction techniques may be applied: principal component analysis (PCA), truncated singular value decomposition, kernel PCA, t-distributed stochastic neighbor embedding, auto-encoder neural network, or any other unsupervised or supervised dimension-reduction technique.

After dimensionality reduction an 18 * N dimensional signal is obtained.

This allows the model to be trained on the most important subdimensions of the sensor data.

In the embodiments with less than three inertial sensors 2, the same concatenation operation is performed so as to obtain a 6 * i * N dimensional signal where 0 < i < 3 is the number of inertial sensors 2 used. In this case an equivalent model can be trained on the 6 * i < 18 channels, simply by changing the number of input dimensions for the input layer of the backbone neural network 6, outlined below.

The system 1 further comprises a backbone neural network 6 that in response to receiving as input the multivariate time series, is configured to extract features arranged in a spatial vector.

According to a preferred embodiment, the backbone neural network 6 is a convolutional residual neural network comprising a stack of parametric 1D convolutions.

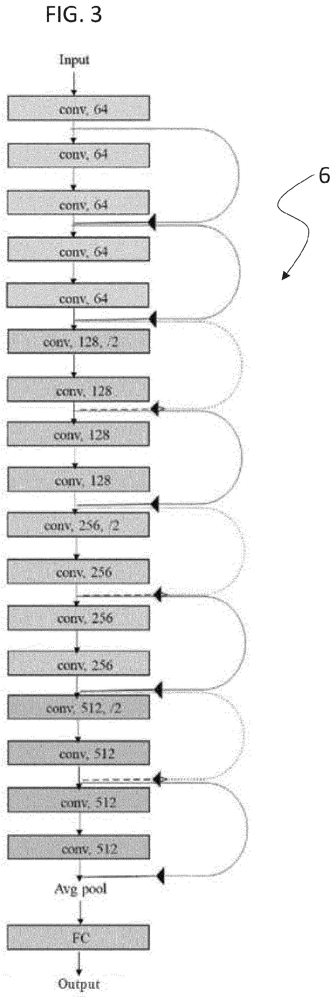

In this preferred embodiment, the structure of the backbone convolutional residual neural network 6 is illustrated in figure 3.

A residual neural network (abbreviated as "ResNet") is a known network that was originally conceived in the field of computer vision image classification. As it is known, ResNet is a stack of parametric 2D convolutions.

In this preferred embodiment of the claimed invention, the residual neural network 6 has 1D convolutions replacing the former 2D convolutions of the ResNet according to the state of the art.

The backbone neural network 6 is configured to map a multivariate time series of dimension 18 * N into a spatial vector of size 512.

In this preferred embodiment, the backbone residual neural network 6 strongly exploits the notable aspect of known ResNet, namely the residual connections between convolutional layers. This aspect allows the network to pass gradients more accurately in the phase of back-propagation in training, avoiding the "vanishing gradient" problem, and allowing the network to be deeper and hence extract richer sets of features from the input. It also smooths the loss function allowing better minima to be found by gradient descent.

In the claimed invention, the backbone neural network 6 is pre-trained using a technique called "SimCLR", that stands for "Simple Framework for Contrastive Learning of Visual Representation".

For a disclosure of this technique, reference is made to the scientific paper *"Simple Framework for Contrastive Learning of Visual Representations"* by Ting Chen et al., ICML'20 Proceedings of the 37th International Conference on Machine Learning, July 2020, Article No.: 149, Pages 1597-1607.

This technique was first developed for images and is now considered a standard in computer vision. The use in the field of the claimed invention has never been disclosed so far.

The backbone neural network 6 can be further fine-tuned on the labelled data, in conjunction with the first, second and third neural networks that will be introduced hereafter.

Using SimCLR pretraining the backbone neural network 6 learns to extract invariant relevant information from the signals acquired by the inertial sensors 2.

SimCLR pretraining applies contrastive loss to a network's representations of two artificially modified, also known as "augmented", versions of the same input datum x. Training a network to minimise this loss allows it to learn the invariant spatial features that represent the data. The distribution of valid augmentations is defined to mimic the onsets that system 1 has to ignore, for example related to the positioning of the sensors, or differences in speed, amplitude, or smoothness of movement. These augmentations shall be random so that each time they are applied to the same signal, they will produce a different output.

The scope of the above-described pre-training is to allow the backbone neural network 6 to learn a robust vectorial representation of the signal, which should ignore noise and irrelevant features present in the sensor measurement (e.g., white or mechanical noise or patient shaking) and uninformative dynamic-related patterns (e.g., how fast or far a movement is executed).

Annotated data are not required. This is very important because it allows the backbone neural network 6 to be trained on large amounts of unannotated, and therefore inexpensive, data.

In a more advanced embodiment, the backbone neural network 6 is configured to perform a similar dimensionality mapping but can extract even richer feature sets by exploiting alternative convolutional, recurrent, LSTM, attention or other neural network architectures, or classical feature-extraction algorithms including decision trees, random forest, gradient boosted forest, support vector machine, k-means clustering, polynomial regression, logistic regression, rules-based kernel filtering (Sobel Filter, Canny Filter, Laplace Filter).

This includes but is not limited to other architectures from image and timeseries feature-extraction, for example ResNeXt, ConvNeXt, ViT, ResNetAutoencoder, which in turn are based on combinations of any of the following architectures: convolutional layers, recurrent layers, LSTM, attention layers, fully connected layers, random decision tree forest, gradient boosted decision tree forest, support vector machine, k-means clustering, polynomial regression, logistic regression or rules-based kernel filtering.

The system 1 further comprises a first neural network 7 configured to detect exercise events in response to the receipt of the extracted features coming from the backbone neural network 6.

In particular, the first neural network 7 is configured to predict a binary classification output, namely a single number that has to be interpreted as the probability of whether the individual is performing an exercise. According to one embodiment, the first neural network 7 is a 2-layer multilayer Perceptron.

Preferably, the first neural network 7 also applies a ReLU (Rectified Linear Unit) activation function.

Alternatively, the first neural network 7 can apply other types of nonlinear activation functions, such as GELU (Gaussian Error Linear Unit) or sigmoid. The first neural network 7 is trained to recognize if the individual is performing a rehabilitation exercise or is engaged in a different activity, like walking or relaxing.

When the first neural network 7 recognizes that the individual is engaged in a rehabilitation exercise, then the second and third neural networks 8, 9 (introduced here below) are activated.

This allows the second and third neural networks 8, 9 to be focused only on the right (exercising) moments. This simplifies greatly their tasks, since they only need to function correctly during the exercises, allowing for a greater robustness.

In machine learning terms, the first neural network 7 aims at increasing the overall precision of the system 1, decreasing the number of false positive repetitions counted.

In order to function correctly the first neural network 7 must recognize the typical pattern of exercises (highly repetitive) from patterns associated with other activities with which the users are typically engaged in between exercises, which might also show a repetitive pattern, like for example walking.

To cover this functionality, supervised learning is employed for teaching to the first neural network 7 to recognize temporal intervals of exercise against temporal intervals of other activities.

The first neural network 7 is trained using a dataset of labelled examples with annotations regarding the temporal segments in which the individuals were performing the exercises. Preferably, a binary cross entropy loss function, which is referred to as $L_A$, is used to tune the parameters of the first neural network 7.

In alternative embodiments other binary loss functions might be applied here to tune the parameters of the first neural network 7, for example focal loss, Kullback-Leibler divergence loss, hinge loss, or any other loss function that is meaningful in this context.

According to alternative embodiments, the first neural network 7 is chosen among other neural network architectures that solve the same task of converting the feature vector from the backbone neural network 6 into a binary classification prediction.

Examples of such alternatives include convolutional layers, recurrent layers, LSTM, attention layers, or any other neural network-based architectures.

In other embodiments the first neural network 7 may be replaced by an alternative feature classification algorithm that is not based on neural networks, for example a support vector machine, classification tree, random forest classifier, gradient boosted classification forest, k-means clustering, or any of the other techniques known in the field.

In practice, the first neural network 7 receives as input the spatial vector from the backbone neural network 6 and outputs a single scalar value denoting the probability of being inside the exercise execution.

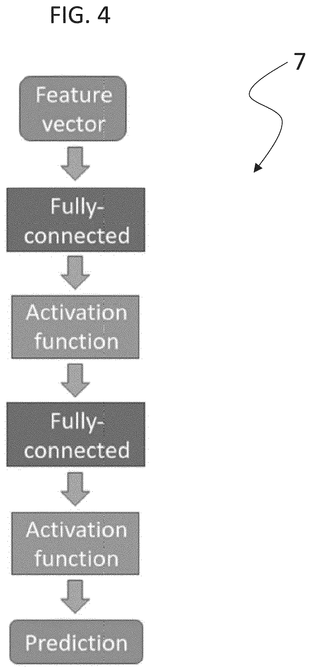

The dataflow of the first neural network 7 is depicted in figure 4.

The system 1 further comprises a second neural network 8 configured to classify the exercise event as belonging to a pool or library of K exercises assigned to the individual by a physician, in response to the receipt of the detected exercise event from the first neural network 7.

According to one embodiment, the second neural network 8 is a 2-layer multilayer Perceptron.

In practice, the second neural network 8 is a multiclass neural network with K distinct probabilistic output units, where the j-th output is interpreted as the probability, estimated by the network, that the individual is performing the j-th exercise.

The dataflow in the second neural network 8 follows the same structure as that of the first neural network 7.

The second neural network 8 is trained using a dataset of labelled examples with annotations encoding the exercise that the individual is performing.

In particular, a categorical-cross-entropy loss function is used to tune the parameters of the second neural network 8 for matching the ground truth annotations. This quantity is referred to as $L_E$.

In alternative embodiments, other multi-class classification loss functions may be used, for example focal loss, Kullback-Leibler divergence loss, hinge loss, cosine similarity, or any other loss function that is meaningful in this context.

In practice, the second neural network 8 is responsible for recognizing the exercise being performed by the individual. Supposing that the exercise library is composed of K possible different exercises, the second neural network 8 outputs a probability vector composed of K entries where the j-th entry denotes the probability that the individual is performing the j-th exercise.

Similar to the first neural network 7, the second neural network 8 receives as input the spatial vector from the backbone neural network 6 and outputs a K dimensional vector that is normalized through a softmax activation function to obtain a valid probability distribution.

According to alternative embodiments, the second neural network 8 may be chosen among other neural network architectures that solve the same task of converting the feature vector from the backbone neural network 6 into a K-dimensional prediction vector.

Examples of such alternatives include convolutional layers, recurrent layers, LSTM, attention layers, or any other neural network-based architectures.

Similar to the first neural network 7, the second neural network 8 may be replaced by an alternative feature multiclass classification algorithm that is not based on neural networks, for example a support vector machine, classification tree, random forest classifier, gradient boosted classification forest, k-means clustering, or any of the other techniques known in the field. The system 1 further comprises a third neural network 9 that, in response to the identification of a correct exercise event by the second neural network 8 and based on the features extracted by the backbone neural network 6, is configured to count repetitions of said identified exercise.

In fact, a complex rehabilitation exercise is decomposed into smaller movements (here called "phases") units consisting of specific coherent movements of one or more limbs.

In this context, a specific coherent movement can be defined as a movement from a rest position to a position of maximal displacement and back to rest, where "rest, movement towards maximal displacement, movement back to the rest position" are the small movement units also called "phases". Coherent movements are movements performed continuously without pauses in between.

Decomposing the exercise in the aforementioned way allows to create a simple finite state machine that can be used for counting an exercise's repetitions.

In a single-phased exercise, the end of a repetition occurs when the unique phase composing the exercise ends. Thus, the repetition counter is incremented by 1.

In a two-phased exercise, the end of a repetition occurs when the two phases composing the exercise end.

In an N-phased exercise, the end of a repetition occurs after N consecutive phases of the exercise are successfully completed by the individual.

Given the periodicity of rehabilitation exercises, a half of a period of a periodic function is used to model the exercise phase (e.g., sin(t) for t in [0, pi]).

In this context, this function is called phase indicator function f(t), with the following properties:

- f(t) is in the range [0, 1];
- f(t) = 0 if the subject is not performing the exercise;
- with Tb denoting the beginning of an exercise phase and Te denoting the end of the phase, f(Tb) = f(Te) = 0;
- with Tm denoting the middle point of the phase, where Tb < Tm < Te, it is required that f(Tm) = 1;
- f(t) is monotonically increasing in [Tb, Tm[;
- f(t) is monotonically decreasing in [Tm, Te[.

According to one embodiment of the invention, the phase indicator function f(t) is implemented as a smoothed sawtooth function, that is smoothed through a gaussian kernel.

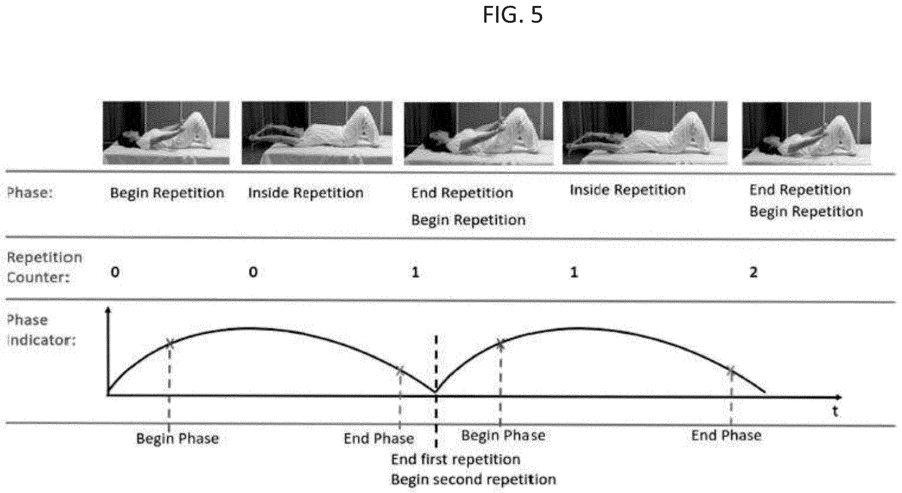

In a first example, illustrated in figure 5, a subject laying down performs an extension of its upper limbs. The phase indicator is 0 in the initial position, when the subject lays both arms on its thighs; the phase indicator increases to reach 1 at the maximum extension of its upper limbs, afterwards the phase indicator decreases till 0 when the subject reaches the initial position again: at that point, the repetition counter increases by 1 unit.

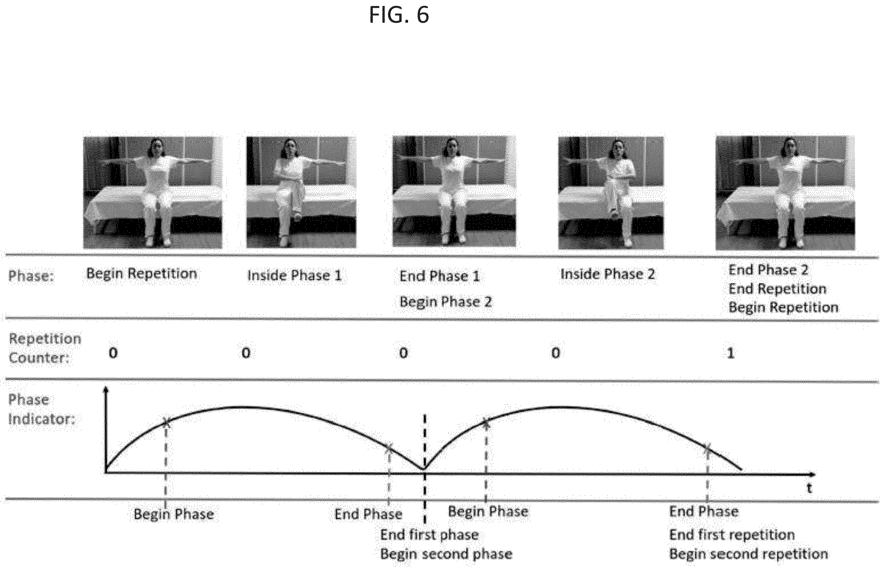

In a second example, illustrated in figure 6, the subject performs a 2-phased exercise: while sitting on a support, the subject starts with the arms spread outwards; during the first exercise phase the subjects moves the left arm on its knee and then retraces the movement to the initial position; during the second exercise phase the subject moves the right arm on its knee and then retraces.

The repetition counter is incremented of 1 unit after both phases are completed.

In addition, it must be noticed that the subject might have approached the same exercise by inverting the phase order (i.e., moving the right arm on its knee before moving the left arm) yielding a still valid repetition.

Exercise complexity has been defined according to five difficulty levels. Each level has been described considering the following criteria:

- exercise pattern: Bilateral Symmetric (BS), Bilateral Alternated (BA), Mono-lateral (ML), Static (ST);
- exercise starting position (comfortable or uncomfortable, e.g., lying down)
- number of limbs involved in the movement;
- number of joints involved in the movement.

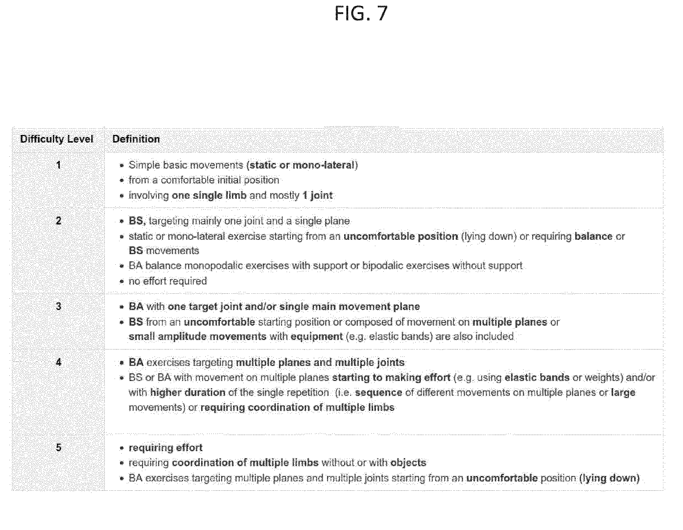

Exercise complexity definition is summarized in the table of figure 7.

In practice, the third neural network 9 receives as input the spatial vector from the backbone neural network 6 and outputs a single scalar value which is a prediction of the phase indicator function.

According to one embodiment, the third neural network 9 is a 2-layer multilayer Perceptron that produces a continuous regression of the phase indicator function.

According to alternative embodiments, the third neural network 9 may be chosen among other neural network architectures that solve the same task of converting the feature vector from the backbone neural network 6 into a regression of the phase indicator function.

Examples of such alternatives include convolutional layers, recurrent layers, LSTM, attention layers, or any other neural network-based architectures.

In further alternative embodiment, the third neural network 9 may be replaced by an alternative continuous regression algorithm that is not based on neural networks, for example polynomial regression, random forest regressor, gradient boosted regression forest, or any of the other techniques known in the field.

The third neural network 9 subsequently applies in post-processing a Finite State Machine (FSM) to the predicted value of the phase indicator function over time. This FSM acts as a counting function that counts the number of phases that have been enacted of a single repetition.

The third neural network 9 is trained end-to-end with annotated data to solve a regression problem, where the loss $L_c$ is a distance function between the network output and the ground-truth phase indicator. In one embodiment this distance function is the mean squared error between the prediction and the true phase indicator function f(t).

In an alternative embodiment this loss function may be replaced by other suitable distance functions, including but not limited to the cross-entropy loss on the interval [0,1], smoothed mean absolute error, log hyperbolic cosine loss, quantile loss, or concordance correlation coefficient loss. After this the post-processing FSM is applied, and outputs a value of 0, except in the moment when a repetition is successfully completed, whereupon it outputs a single 1.

The sum of these outputs of the FSM is the real-time predicted count of exercise repetitions.

The dataflow of the third neural network 9 is depicted in figure 6.

In summary, for the third neural network 9 the following logic is implemented:

- each exercise of the library is decomposed in a sequence of "coherent atomic movements" (phases), which constitutes a repetition;
- during the data acquisition, the start/end of each phase composing the exercise, as well as the start/end of each repetition are labelled;
- each phase is modeled with the phase indicator function as described above;
- the third neural network 7 solves a regression problem from the input sensor sequence to the ground-truth phase indicator.
- the FSM converts the phase indicator regression value into a real-time discrete count of the number of exercise repetitions.

In view of what is described above, the third neural network 9 creates a nonlinear transformation mapping from the input space of the feature vector extracted by the backbone neural network 6 to a one-dimensional space, in which the phase of the exercise is represented by a regular curve.

The advantage of this transformation is that counting the repetitions during inference is much easier.

Notwithstanding the specific algorithm for counting in inference, it is sufficient that the third neural network 9 is able to count phase transitions of the transformed signal and divide by the number of phases that make up a repetition.

The phase indicator function may be conceived as an abstraction layer, through which different exercises with different patterns are transformed to the same patterns, which definitively depends only on the fraction of completion of the repetition.

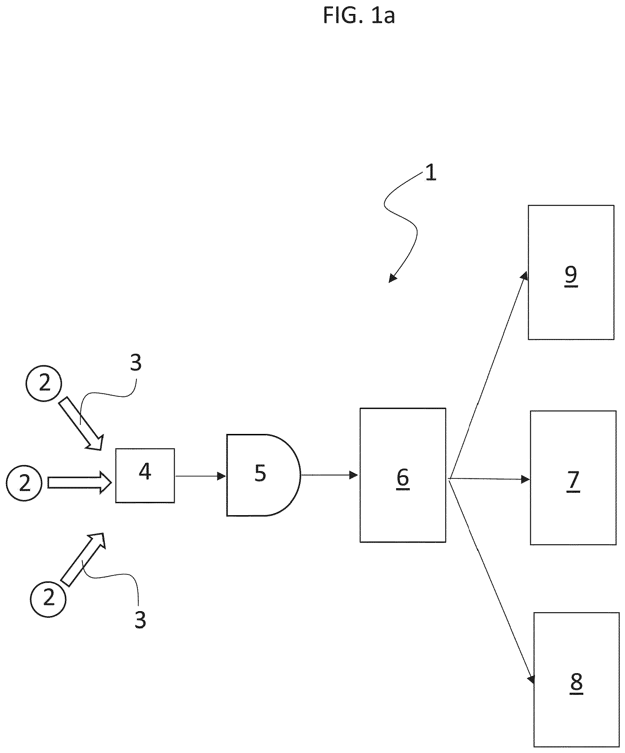

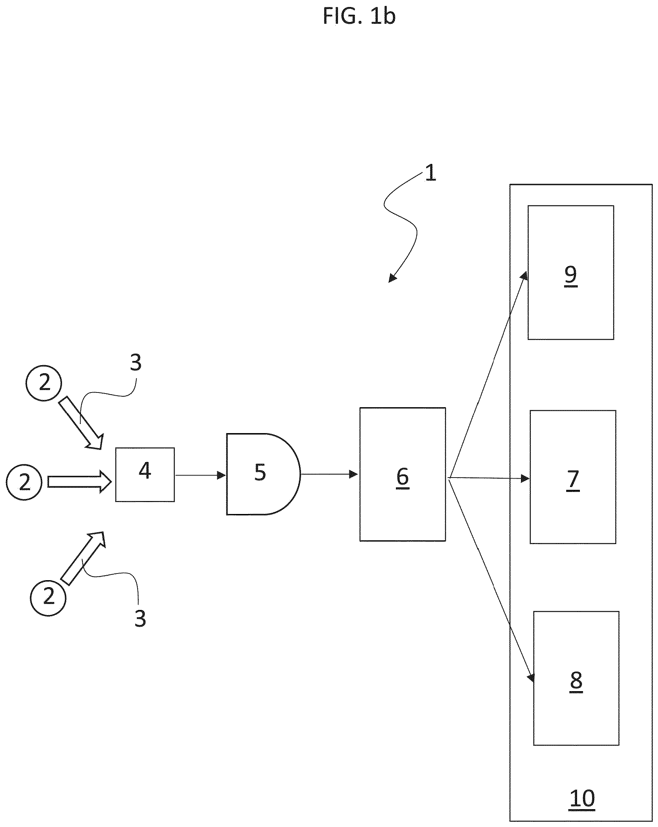

According to one embodiment, the three neural networks 7, 8, 9 could be united in one functional block 10 sharing a single backbone neural network 6. This is shown in the embodiment of fig. 1b.

In other embodiments the three neural networks 7, 8 and 9 may retain distinct backbone neural networks 6, or two of them may share a backbone neural network 6 and the other have a distinct backbone neural network 6. In other embodiments the backbone neural network 6 for each of the three neural networks 7, 8 and 9 may even possess an architecture distinct from that of the others, for example other convolutional, attention, LSTM, recurrent architectures, or indeed any suitable neural network feature extraction architecture.

During the fine-tuning, all the weights are updated for all the units of the system including the backbone neural network 6. To achieve this goal, the neural networks are instructed to minimize a loss composed as:

$$ L = c_A * L_A + c_E * L_E + c_C * L_C + c_{DA} * L_{DA,} $$

which is minimized by means of gradient descent.

$L_{DA}$ stands for domain adaptation loss and will be introduced below.

The coefficients $c_A$, $c_E$, $c_C$, and $c_{DA}$ are used to scale the relative importance of each loss term, which is very important for this kind of multi-objective minimization problem, since the losses might be competing with each other and live on different scales.

It is noted the coefficients might be adaptive and might be searched with a cross-validated grid search to set them to their optimal value.

The domain adaptation loss is a key ingredient introduced here for improving the performance of the system on complex exercises and allowing to make use of large amounts of unlabeled (and therefore unexpensive) data, collected while new user use the system.

Moreover, the domain adaptation loss accounts for the fact that labelled data acquired from patients is expensive and might not be possible to collect for privacy related issues.

The technical details and theoretical foundation of domain adaptation techniques are disclosed in the scientific paper "A theory of learning from different domains" by Shai Ben-David et al, retrievable with open access at https://link.springer.com/content/pdf/10.1007/s10994-009-5152-4.pdf.

For example, the following databases are available:

- a labeled database $D_A$ of samples collected on the exercises of a pool of subjects A;
- an unlabeled database $D_B$ of samples collected on the exercises of a different pool of subjects B.

The subjects in the pool A, on which supervised learning is performed, might not be completely representative of the pool B, to which it is desired to apply the inference.

An example is the subjects belonging to the two pools having different degrees of movement impairment.

Then the domain adaptation loss is used to find a trade-off so that the losses of the activity, exercise and counting units are minimized without relying only on features which are specific to the samples in the database $D_A$.

The domain adaptation loss acts only on the features extracted by the backbone and forces them to share the same probability distribution between $D_A$ and $D_B$.

The output of the system 1 may be forwarded to a device equipped with a monitor that shows in real-time to the user the output of inference method described below (for example the number of repetitions executed, the correctness of the ongoing exercise or possible issues related to the exercise execution).

Since the system 1 comprises a multi-headed neural network, its functionality is split into two distinct phases: a learning phase, where the parameters of the system are tuned on the data, and an inference phase where the outcomes of the system are suitably presented to the subject during its exercise sessions.

The claimed system 1 tracks the performance of the subject over time, by tracking a quality score given to each exercise session it performs.

The quality score is a number bounded between 0 and 1, where 1 means a perfect execution.

Assuming that an exercise session is composed of a series of exercises $\{E(1), ..., E(K)\}$, the session score is computed as the average of the score given to the singular exercises: SessionScore = Average([Score(1), ..., Score(K)]).

For a single exercise E(j) for which the therapist has assigned the patient to perform N repetitions, the score is computed in the following way:

- for each correctly detected repetition, the average of the score provided by the exercise unit is computed;
- for each correctly detected repetition, the sum of the score obtained in the previous point is computed, and divided by N. The value obtained this way is in the range [0, 1[ and penalizes missing repetitions.

In particular, the learning phase can be made incremental, and newly acquired data can improve the performances of the system over time.

The learning phase of the system is composed of two sub-phases:

- a pre-training, which affects only the backbone neural network 6;
- a common fine-tuning phase, in which the units are specialized to execute their specific task.

The learning phase has already been introduced above, together with the disclosure of the system.

Here follows the description of the inference phase.

A computer-implemented method for counting repetitions of physical exercises by an individual is addressed with number 100.

The method 100 comprises at least the following steps:

- arranging at least one inertial sensor in a body part of the individual (step indicated with 101);
- sampling signals that are representative of biometric and/or medical parameters of the individual using the inertial sensors so as to obtain sampled data (step indicated with 102);
- normalizing the sampled data (step indicated with 103);
- concatenating the normalized sampled data so as to obtain a single flow of data in the form of a multivariate time series (step indicated with 104);
- extracting features from the multivariate time series by using a backbone neural network 6 that has been pre-trained by SimCLR and fine-tuned (step indicated with 105);
- detecting exercise events in response to the receipt of the extracted features by using a first neural network 7 (step indicated with 106);
- in response to an exercise event detected by the first neural network and based on the features extracted by the backbone neural network 6, identifying the exercise event out of a pool of K different exercises by means of a second neural network 8 (step indicated with 107);
- in response to the identification of an exercise event by the second neural network 8 and based on the features extracted by the backbone neural network 6, counting repetitions of the identified exercise by means of a third neural network 9 that has been trained to decompose each exercise in one or more temporal segments or continuous phases and includes a post-processing step that converts this continuous prediction into a discrete count of the number of executed exercise repetitions (step indicated with 108).

The inertial sensors 2 are arranged on multiple points of the body of an individual and start sampling signals that are representative of biometric and/or medical parameters.

The sampled data are represented as the channels 3 and provided as input to the normalization unit 4, where they arrived in a chronological order.

The normalization unit 4 applies a normalization formula to the sampled data. The normalized data are stored in the buffer within the normalization unit 4.

The buffer is preferably a circular buffer, whose dimension is chosen depending on the specific exercise performed by the individual: shorter exercises require a shorter buffer, whereas longer exercises require a longer buffer.

When the buffer of the normalization unit 4 is full, then it is started the transmission of the stored normalized data from the buffer to the fusion unit 5.

This operation is performed with a stride of m seconds (i.e., each m seconds the updated data contained in the buffer is forwarded to the units downstream for inference).

The exact value of the stride is chosen considering the trade-off between performances, responsiveness, and resource requirements constraints.

The fusion unit 5 provides a multivariate time series that is obtained by at least a concatenation of the normalized data coming from the normalization unit 4.

The multivariate time series are fed to the backbone neural network 6 that extracts features arranged in a spatial vector of size 512.

In the preferred embodiment, the backbone neural network 6 is a convolutional ResNet configured to map a multivariate time series of dimension 18 * N into a spatial vector of size 512.

The spatial vector is sent to the three neural networks 7, 8, 9.

In practice, the first neural network 7 acts as an enabler for the second and third neural networks 8 and 9.

If a non-exercise is recognized, the second and third neural networks 8, 9 are indeed inhibited. This results in a more robust system.

If an exercise is detected by the first neural network 7, then the second and third neural networks 8, 9 are enabled.

The second neural network 8 processes the features extracted by the backbone neural network 6 and assesses the correctness of the exercise executed by the individual.

Possible applications of the second neural network 8 are:

- estimating the quality of exercises execution by signaling correct and mistaken units of motion;
- stopping the user as soon as unexpected and possibly detrimental movements are detected;
- storing anomalous behaviors for further (offline) analysis;
- checking the posture during the exercise execution;
- estimating/quantifying deviation from healthy reference execution.

The third neural network 9 processes the features extracted by the backbone neural network 6 and outputs the number of correctly executed repetitions. The third neural network 9 has the following properties:

- supports exercises composed of multiple phases and involving the movement of potentially different limbs, with monolateral and bilateral movements;
- is able to count exercise repetitions performed at different speed.

The specific counting algorithm applied by the third neural network 9 comprises:

- applying a smoothing low-pass filter to remove spurious oscillations and to reduce the amount of noise in the indicator function;
- initializing a phase counter to N, the number of phases composing the exercise;
- repeating the following cycle till the phase counter is decreased to zero:

  1. initializing the Finite State Machine (FSM) with state $S = S_{init}$
  2. as soon as the phase indicator function satisfies certain predefined logical conditions, applying a state transition to the state which is set to phase: $S = S_{phase}$;
  3. as soon as the phase indicator function satisfies certain other logical conditions, resetting the state to $S = S_{init}$ (i.e., it is considered that an exercise phase has just been completed) and decreasing the phase counter of one unit.

- Once the phase counter reaches 0, increasing the number of detected repetitions by 1 unit.

In one preferred embodiment, the smoothing filter stage comprises a Butterworth low-pass filter applied to the predicted phase indicator function. In alternative embodiments, the smoothing filter stage can use other algorithms to perform the filtering, including but not limited to adaptive filtering, Kalman filtering, or a neural network denoising autoencoder.

In one preferred embodiment the FSM changes state based on the smoothed predicted phase indicator function exceeding or subceeding certain predefined thresholds.

In alternative embodiments, the FSM changes state based on one or more conditions determined from the smoothed predicted phase indicator function, including but not limited to conditions on: the gradient (first derivative w.r.t time), higher order derivatives w.r.t time, or thresholds that vary based on other values such as the current phase counter or repetition counter.

The characteristics of the system and method for identifying, analysing, and counting repetitions of physical exercises by an individual, according to the present invention, are clear, as are the advantages.

The claimed invention helps reducing the amount of data needed for training because many different exercises usually share common sub-movements. By feeding the system with samples from all types of exercises, the system can effectively learn the simplest movements, regardless of the exercises they belong to, therefore reducing drastically the data required for training.

The claimed invention allows to detect even complex exercises, composed of a sequence of movements on different axes in several phases, also involving more limbs (e.g., bilateral exercises), usually adopted into clinical practice as part of rehabilitation protocols.

Thus, in contrast with prior art solutions, often requiring the definition of a custom model for each exercise, the claimed invention is more flexible since it allows a quick and easy addition of new exercises.

In fact, including new exercises in the library does not require to collect huge amounts of data for training.

The claimed invention works properly both for healthy and impaired subjects and can be used both in the clinic and at home.

In particular, the system can assist patients in performing physical rehabilitation programs without the physical presence of a physician.

In an application of the claimed invention, exercise definition also includes ADLs, which are "Activities of Daily Living" that refer to people's daily activities (such as, for instance, personal hygiene or grooming, dressing, toileting, transferring or ambulating, eating, etc). ADLs may indeed be split in phases, so patients may be monitored in their improvement in performing these activities.

The system is able to identify, analyse and count repetitions of physical exercises executed by the subjects and, from this, it may be used to provide real-time feedback to the subjects, for example assessing the correctness of the exercises and tracking their improvements.

**Claims**

1. A system (1) for identifying, analysing, and counting repetitions of physical exercises by an individual, the system (1) comprising:

   - at least one inertial sensor (2) that is wearable by the individual and is configured to sample corresponding signals representative of biometric and/or medical parameters of the individual so as to obtain sampled data;
   - a normalization unit (4) configured to receive the sampled data from said at least one inertial sensor (2) and to normalize them so as to obtain normalized data;
   - a fusion unit (5) configured to receive sets of normalized data from the normalization unit (4) and to output a single flow of data in the form of a multivariate time series that is obtained by at least a concatenation of the normalized data coming from the normalization unit (4);
   the system being **characterized in that** it further comprises:

   - a backbone neural network (6) that, in response to receiving as input the multivariate time series, is configured to extract features arranged in a spatial vector, said backbone neural network (6) having been pre-trained by a Simple Framework for Contrastive Learning of Visual Representation, SimCLR, technique;
   - a first neural network (7) configured to detect exercise events in response to the receipt of the extracted features coming from the backbone neural network (6), said first neural network (7) having been trained to detect exercise events using a dataset of labelled examples with annotations regarding temporal segments in which individuals were performing the exercises, wherein a binary cross entropy loss function $L_A$ is used to tune parameters of the first neural network (7);
   - a second neural network (8) that, in response to an exercise event detected by the first neural network (7) and based on the features extracted by the backbone neural network (6), is configured to identify the exercise event out of a pool of K different exercises the individual may be assigned, said second neural network (8) having been trained to identify the exercises using a dataset of labelled examples with annotations encoding the exercise that the individual is performing, a categorical-cross-entropy loss function $L_E$ being used to tune parameters of the second neural network (8) for matching ground truth annotations;
   - a third neural network (9) that, in response to the identification of an exercise event by the second neural network (8) and based on the features extracted by the backbone neural network (6), is configured to count repetitions of said identified exercise, said third neural network (9) having been trained to decompose each exercise into one or more timesegments or phases and apply a subsequent discretizing counting function to said phases, said third neural network (9) having been trained end-to-end with annotated data to solve a regression problem, where loss Lc is a distance function between the third neural network output and a ground-truth phase indicator;

- a version of the backbone neural network (6) that has been adapted to the tasks of binary exercise classification, multiclass exercise classification and regression of the phase indicator function, by fine-tuning the backbone neural network (6) combined with the three neural networks (7), (8) and (9) on labelled data to achieve the aforementioned tasks, wherein during the fine-tuning the neural networks (6, 7, 8, 9) are instructed to minimize a loss composed as: $L = c_A * L_A + c_E * L_E + c_C * Lc + c_{DA} * L_{DA}$, which is minimized by means of gradient descent, where $L_{DA}$ stands for domain adaptation loss *and* $c_A$, $c_E$, cc, and $c_{DA}$ are coefficient used to scale corresponding loss terms,

wherein the backbone neural network (6) is a convolutional residual neural network (6) comprising a stack of parametric 1D convolutions, said backbone neural network (6) being configured to map a multivariate time series of dimension 18 * N into a spatial vector of size 512.

2. The system (1) according to claim 1, wherein said at least one inertial sensor (2) is a 6-axis inertial sensor comprising an accelerometer and a gyroscope.

3. The system (1) according to any one of the preceding claims, further comprising a plurality of channels (3) for transmitting sampled data from the inertial sensor (2) to the normalization unit (4), each of said channels (3) being configured to receive and transmit the sampled data in a chronological order.

4. The system (1) according to any one of the preceding claims, wherein the normalization unit (4) comprises a buffer to store normalized data.

5. The system (1) according to any one of the preceding claims, wherein each of the first neural network (7), the second neural network (8) and the third neural network (9) is a 2-layer multi-layer Perceptron.

6. The system (1) according to any one of the claims 1 to 4, wherein the first neural network (7), second neural network (8) and/or third neural network (8) is chosen among a combination of any of the following architectures: convolutional layers, recurrent layers, LSTM, attention layers, fully connected layers, random decision tree forest, gradient boosted decision tree forest, support vector machine, k-means clustering, polynomial regression, logistic regression.

7. The system (1) according to any one of the preceding claims, wherein each phase of an exercise is modeled with a phase indicator function f(t) having the following properties:

   • f(t) is in the range [0, 1];
   • f(t) = 0 if the individual is not performing the exercise;
   • with Tb denoting the beginning of the exercise phase and Te denoting the end of the exercise phase, f(Tb) = f(Te) = 0;
   • with Tm denoting the middle point of the exercise phase, where Tb < Tm < Te, f(Tm) = 1;
   • f(t) is monotonically increasing in [Tb, Tm[;
   • f(t) is monotonically decreasing in [Tm, Te[.

8. The system (1) according to claim 7, wherein the phase indicator function f(t) is implemented as a smoothed sawtooth function, that is smoothed through a gaussian kernel.

9. The system (1) according to claim 8, wherein the third neural network (9) includes a Finite State Machine, FSM, that is configured to accept a smoothed regression prediction of said phase indicator function f(t) and to output either the numeric value "1" when a phase of a repetition of an exercise has been completed or the numeric value "0" in any other case, based on when said smoothed regression prediction satisfies certain predefined logical conditions that are a combination of any of the following: thresholds on the function value, thresholds on the gradient, thresholds on the second or higher-order derivatives, adaptive thresholds based on the state and/or outputs of the FSM.

10. A computer-implemented method (100) for identifying, analysing, and counting repetitions of physical exercises by an individual using the system (1) according to any one of the preceding claims, the method (100) comprising the following steps:

- arranging at least one inertial sensor in a body part of the individual (101);
- sampling signals that are representative of biometric and/or medical parameters of the individual using said at least one inertial sensor so as to obtain sampled data (102);
- normalizing the sampled data (103);
- concatenating the normalized sampled data so as to obtain a single flow of data in the form of a multivariate time series (104);
- extracting features from the multivariate time series by using the backbone neural network of said system, said backbone neural network having been pre-trained by a Simple Framework for Contrastive Learning of Visual Representation, SimCLR, technique (105);
- detecting exercise events in response to the receipt of the extracted features by using the first neural network of said system (106);
- in response to an exercise event detected by the first neural network and based on the features extracted by the backbone neural network, identifying the exercise event out of a pool of K different exercises by means of the second neural network of said system (107);
- in response to the identification of an exercise event by the second neural network and based on the features extracted by the backbone neural network, counting repetitions of said identified exercise by means of the third neural network of said system, said third neural network having been trained to decompose each exercise in one or more time segments or phases (108).

11. The method (100) according to claim 10, wherein each phase of an exercise is modeled with a phase indicator function $f(t)$ having the following properties:

- $f(t)$ is in the range $[0, 1]$;
- $f(t) = 0$ if the individual is not performing the exercise;
- with $T_b$ denoting the beginning of the exercise phase and $T_e$ denoting the end of the exercise phase, $f(T_b) = f(T_e) = 0$;
- with $T_m$ denoting the middle point of the exercise phase, where $T_b < T_m < T_e$, $f(T_m) = 1$;
- $f(t)$ is monotonically increasing in $[T_b, T_m[$;
- $f(t)$ is monotonically decreasing in $[T_m, T_e[$.

12. The method (100) according to claim 11, wherein the step of counting the repetitions (108) comprises the following sub-steps:

- applying a smoothing low-pass filter to remove spurious oscillations and to reduce the amount of noise in the phase indicator function;
- initializing a phase counter to N, that is the number of phases composing the exercise;
- until the phase counter is zero, repeating a cycle comprising:

    - initializing a Finite State Machine of the third neural network to an initial state;
    - as soon as the filtered phase indicator function satisfies certain logical conditions, applying a state transition to the state which is set to a state exercise, otherwise it is left unchanged;
    - as soon as the filtered phase indicator function satisfies other logical conditions, resetting the state to the initial state and decreasing the phase counter of one unit;

- when the phase counter reaches zero, increasing the number of detected repetitions by one unit and resetting the phase counter to N.

13. Computer program having instructions which, when executed by a computer device, cause the computing device to perform the method according to any one of claims 10 to 12.

**Patentansprüche**

1. System (1) zur Identifizierung, Analyse und Zählung von Wiederholungen körperlicher Übungen durch eine Person, wobei das System (1) umfasst:

- mindestens einen Trägheitssensor (2), der von der Person getragen werden kann und so ausgelegt ist, dass er entsprechende Signale abtastet, die für biometrische und/oder medizinische Parameter der Person repräsentativ sind, um abgetastete Daten zu erhalten;

- eine Normalisierungseinheit (4), die ausgelegt ist, um die abgetasteten Daten von dem mindestens einen Trägheitssensor (2) zu empfangen und zu normalisieren, um normalisierte Daten zu erhalten;

- eine Fusionseinheit (5), die ausgelegt ist, um Sätze von normalisierten Daten von der Normalisierungseinheit (4) zu empfangen und einen einzelnen Datenfluss in Form einer multivariaten Zeitreihe auszugeben, die durch mindestens eine Verkettung der von der Normalisierungseinheit (4) kommenden normalisierten Daten erhalten wird;

wobei das System **dadurch gekennzeichnet ist, dass** es ferner Folgendes umfasst:

- ein neuronales Backbone-Netzwerk (6), das als Reaktion auf das Empfangen der multivariaten Zeitreihe als Eingabe ausgelegt ist, um Merkmale zu extrahieren, die in einem räumlichen Vektor angeordnet sind, wobei das neuronale Backbone-Netzwerk (6) durch eine SimCLR-Technik (Simple Framework for Contrastive Learning of Visual Representation) vortrainiert wurde;

- ein erstes neuronales Netzwerk (7), das ausgelegt ist, um Übungsereignisse als Reaktion auf den Empfang der extrahierten Merkmale aus dem neuronalen Backbone-Netzwerk (6) zu erkennen, wobei das erste neuronale Netzwerk (7) trainiert wurde, um Übungsereignisse unter Verwendung eines Datensatzes von etikettierten Beispielen mit Anmerkungen betreffend zeitliche Segmente, in denen Personen die Übungen durchführten, zu erkennen, wobei eine binäre Kreuzentropieverlustfunktion $L_A$ verwendet wird, um Parameter des ersten neuronalen Netzwerks (7) abzustimmen;

- ein zweites neuronales Netzwerk (8), das als Reaktion auf ein von dem ersten neuronalen Netzwerk (7) erkanntes Übungsereignis und auf der Grundlage der von dem neuronalen Backbone-Netzwerk (6) extrahierten Merkmale ausgelegt ist, um das Übungsereignis aus einem Pool von K verschiedenen Übungen zu identifizieren, die der Person zugewiesen werden können, wobei das zweite neuronale Netzwerk (8) trainiert wurde, um die Übungen unter Verwendung eines Datensatzes von etikettierten Beispielen mit Anmerkungen zu identifizieren, die die Übung kodieren, die die Person durchführt, wobei eine kategoriale Kreuzentropieverlustfunktion $L_E$ verwendet wird, um Parameter des zweiten neuronalen Netzwerks (8) abzustimmen, um Grundwahrheitsanmerkungen abzugleichen;

- ein drittes neuronales Netzwerk (9), das als Reaktion auf die Identifizierung eines Übungsereignisses durch das zweite neuronale Netzwerk (8) und auf der Grundlage der durch das neuronale Backbone-Netzwerk (6) extrahierten Merkmale ausgelegt ist, um Wiederholungen der identifizierten Übung zu zählen, wobei das dritte neuronale Netzwerk (9) trainiert wurde, um eine jede Übung in ein oder mehrere Zeitsegmente oder Phasen zu zerlegen und eine nachfolgende diskretisierende Zählfunktion auf die Phasen anzuwenden, wobei das dritte neuronale Netzwerk (9) Ende-zu-Ende mit annotierten Daten trainiert wurde, um ein Regressionsproblem zu lösen, wobei der Verlust Lc eine Abstandsfunktion zwischen dem Ausgang des dritten neuronalen Netzwerks und einem Ground-Truth-Phasenindikator ist;

- eine Version des neuronalen Backbone-Netzwerks (6), die an die Aufgaben der binären Übungsklassifizierung, der Multiklassenübungsklassifizierung und der Regression der Phasenindikatorfunktion angepasst wurde, indem das neuronale Backbone-Netzwerk (6) in Kombination mit den drei neuronalen Netzwerken (7), (8) und (9) auf markierte Daten feinabgestimmt wird, um die oben genannten Aufgaben zu erfüllen, wobei die neuronalen Netzwerke (6, 7, 8, 9) während der Feinabstimmung angewiesen werden, einen Verlust zu minimieren, der sich wie folgt zusammensetzt: $L = C_A * L_A + C_E * L_E + C_C * Lc + C_{DA} * L_{DA}$, welches durch Gradientenabstieg minimiert wird, wobei $L_{DA}$ für Domänenadaptionsverlust steht *und* $C_A$, $C_E$, $C_C$, und $C_{DA}$ Koeffizienten sind, die zur Skalierung entsprechender Verlustterme verwendet werden,

wobei das neuronale Backbone-Netzwerk (6) ein neuronales Faltungsrestnetzwerk (6) ist, das einen Stapel parametrischer 1D-Faltungen umfasst, wobei das neuronale Backbone-Netzwerk (6) ausgelegt ist, um eine multivariate Zeitreihe der Dimension 18 * N in einen räumlichen Vektor der Größe 512 abzubilden.

2. System (1) nach Anspruch 1, wobei der mindestens eine Trägheitssensor (2) ein 6-Achsen-Trägheitssensor ist, der einen Beschleunigungsmesser und ein Gyroskop umfasst.

3. System (1) nach einem der vorhergehenden Ansprüche, ferner umfassend eine Vielzahl von Kanälen (3) zum Übertragen von abgetasteten Daten von dem Trägheitssensor (2) zu der Normalisierungseinheit (4), wobei ein jeder der Kanäle (3) ausgelegt ist, um die abgetasteten Daten in einer chronologischen Reihenfolge zu empfangen und zu übertragen.

**4.** System (1) nach einem der vorhergehenden Ansprüche, wobei die Normalisierungseinheit (4) einen Puffer zum Speichern normalisierter Daten umfasst.

**5.** System (1) nach einem der vorhergehenden Ansprüche, wobei das erste neuronale Netzwerk (7), das zweite neuronale Netzwerk (8) und das dritte neuronale Netzwerk (9) jeweils ein zweischichtiges mehrschichtiges Perceptron ist.

**6.** System (1) nach einem der Ansprüche 1 bis 4, wobei das erste neuronale Netzwerk (7), das zweite neuronale Netzwerk (8) und/oder das dritte neuronale Netzwerk (8) aus einer Kombination einer beliebigen der folgenden Architekturen ausgewählt ist: Faltungsschichten, wiederkehrende Schichten, LSTM, Aufmerksamkeitsschichten, vollständig verbundene Schichten, Zufallsentscheidungsbaumwald, gradientenverstärkte Entscheidungsbaumwald, Unterstützungsvektormaschine, k-Mittel-Clustering, polynomiale Regression, logistische Regression.

**7.** System (1) nach einem der vorhergehenden Ansprüche, wobei eine jede Phase einer Übung mit einer Phasenindikatorfunktion f(t) mit den folgenden Eigenschaften modelliert wird:

- f(t) im Bereich [0, 1] liegt;
- f(t) = 0, wenn die Person die Übung nicht durchführt;
- wobei Tb den Beginn der Übungsphase bezeichnet und Te das Ende der Übungsphase bezeichnet, f(Tb) = f(Te) = 0;
- wobei Tm den Mittelpunkt der Übungsphase bezeichnet, wobei Tb < Tm < Te, f(Tm) = 1;
- f(t) monoton in [Tb, Tm[ ansteigend ist;
- f(t) monoton [Tm, Te[ fallend ist.

**8.** System (1) nach Anspruch 7, wobei die Phasenindikatorfunktion f(t) als geglättete Sägezahnfunktion implementiert ist, die durch einen Gauß-Kern geglättet ist.

**9.** System (1) nach Anspruch 8, wobei das dritte neuronale Netzwerk (9) eine Finite-State-Maschine, FSM, beinhaltet, die dazu ausgelegt ist, eine geglättete Regressionsvorhersage der Phasenindikatorfunktion f(t) zu akzeptieren und entweder den numerischen Wert "1" auszugeben, wenn eine Phase einer Wiederholung einer Übung abgeschlossen wurde, oder den numerischen Wert "0" in einem jeden anderen Fall, basierend darauf, wenn die geglättete Regressionsvorhersage bestimmte vordefinierte logische Bedingungen erfüllt, die eine Kombination aus einem der Folgenden sind: Schwellenwerte für den Funktionswert, Schwellenwerte für den Gradienten, Schwellenwerte für die Ableitungen zweiter oder höherer Ordnung, adaptive Schwellenwerte basierend auf dem Zustand und/oder Ausgaben der FSM.

**10.** Computerimplementiertes Verfahren (100) zur Identifizierung, Analyse und Zählung von Wiederholungen körperlicher Übungen durch eine Person unter Verwendung des Systems (1) nach einem der vorhergehenden Ansprüche, wobei das Verfahren (100) die folgenden Schritte umfasst:

- Anordnen mindestens eines Trägheitssensors in einem Körperteil der Person (101);
- Abtasten von Signalen, die für biometrische und/oder medizinische Parameter der Person repräsentativ sind, unter Verwendung des mindestens einen Trägheitssensors, um abgetastete Daten (102) zu erhalten;
- Normalisieren der abgetasteten Daten (103);
- Verketten der normalisierten abgetasteten Daten, um einen einzelnen Datenfluss in Form einer multivariaten Zeitreihe (104) zu erhalten;
- Extrahieren von Merkmalen aus den multivariaten Zeitreihen unter Verwendung des neuronalen Backbone-Netzwerks des Systems, wobei das neuronale Backbone-Netzwerk durch eine SimCLR-Technik (Simple Framework for Contrastive Learning of Visual Representation (105) vortrainiert wurde;
- Erkennen von Übungsereignissen als Reaktion auf den Empfang der extrahierten Merkmale unter Verwendung des ersten neuronalen Netzwerks des Systems (106);
- als Reaktion auf ein von dem ersten neuronalen Netzwerk erkanntes Übungsereignis und basierend auf den von dem neuronalen Backbone-Netzwerk extrahierten Merkmalen, Identifizieren des Übungsereignisses aus einem Pool von K verschiedenen Übungen mittels des zweiten neuronalen Netzwerks des Systems (107);
- als Reaktion auf die Identifizierung eines Übungsereignisses durch das zweite neuronale Netzwerk und basierend auf den durch das neuronale Backbone-Netzwerk extrahierten Merkmalen, Zählen von Wiederholungen der identifizierten Übung mittels des dritten neuronalen Netzwerks des Systems, wobei das dritte neuronale Netzwerk trainiert wurde, um eine jede Übung in einem oder mehreren Zeitsegmenten oder Phasen (108) zu zerlegen.

11. Verfahren (100) nach Anspruch 10, wobei eine jede Phase einer Übung mit einer Phasenindikatorfunktion f(t) mit den folgenden Eigenschaften modelliert wird:

• f(t) im Bereich [0, 1] liegt;
• f(t) = 0, wenn die Person die Übung nicht durchführt;
• wobei Tb den Beginn der Übungsphase bezeichnet und Te das Ende der Übungsphase bezeichnet, f(Tb) = f(Te) = 0;
• wobei Tm den Mittelpunkt der Übungsphase bezeichnet, wobei Tb < Tm < Te, f(Tm) = 1;
• f(t) monoton in [Tb, Tm[ ansteigend ist;
• f(t) monoton [Tm, Te[ fallend ist.

12. Verfahren (100) nach Anspruch 11, wobei der Schritt zum Zählen der Wiederholungen (108) die folgenden Teilschritte umfasst:

- Anwenden eines glättenden Tiefpassfilters, um störende Schwingungen zu entfernen und die Menge an Rauschen in der Phasenindikatorfunktion zu reduzieren;
- Initialisieren eines Phasenzählers zu N, d.h. der Anzahl der Phasen, aus denen sich die Übung zusammensetzt;
- bis der Phasenzähler Null ist, Wiederholen eines Zyklus, der Folgendes umfasst:

• Initialisieren einer Finite-State-Machine des dritten neuronalen Netzes in einen Ausgangszustand;
• sobald die gefilterte Phasenindikatorfunktion bestimmte logische Bedingungen erfüllt, Anwenden eines Zustandsübergangs auf den Zustand, der auf eine Zustandsübung eingestellt ist, andernfalls wird er unverändert gelassen;
• sobald die gefilterte Phasenindikatorfunktion andere logische Bedingungen erfüllt, Zurücksetzen des Zustands auf den Ausgangszustand und Verringern des Phasenzählers einer Einheit;

- wenn der Phasenzähler Null erreicht, Erhöhen der Anzahl der erkannten Wiederholungen um eine Einheit und Zurücksetzen des Phasenzählers auf N.

13. Computerprogramm, aufweisend Anweisungen, die, wenn sie von einer Computervorrichtung ausgeführt werden, die Computervorrichtung dazu veranlassen, das Verfahren nach einem der Ansprüche 10 bis 12 durchzuführen.

**Revendications**

1. Système (1) d'identification, d'analyse et de comptage de répétitions d'exercices physiques par un individu, le système (1) comprenant :

- au moins un capteur inertiel (2) étant portable par l'individu et configuré pour échantillonner des signaux correspondants représentatifs de paramètres biométriques et/ou médicaux de l'individu afin d'obtenir des données échantillonnées ;
- une unité de normalisation (4) configurée pour recevoir les données échantillonnées dudit au moins un capteur inertiel (2) et pour les normaliser afin d'obtenir des données normalisées ;
- une unité de fusion (5) configurée pour recevoir des ensembles de données normalisées de l'unité de normalisation (4) et pour sortir un flux unique de données sous la forme d'une série temporelle multivariée étant obtenue par au moins une concaténation des données normalisées provenant de l'unité de normalisation (4) ;
le système étant **caractérisé en ce qu'**il comprend en outre :

- un réseau neuronal dorsal (6) qui, en réponse à la réception en entrée de la série temporelle multi-variable, est configuré pour extraire des caractéristiques disposées dans un vecteur spatial, ledit réseau neuronal dorsal (6) ayant été pré-entraîné par une technique de cadre simple pour l'apprentissage contrastif de la représentation visuelle, SimCLR ;
- un premier réseau neuronal (7) configuré pour détecter des activités physiques en réponse à la réception des caractéristiques extraites provenant du réseau neuronal dorsal (6), ledit premier réseau neuronal (7) ayant été entraîné pour détecter des activités physiques à l'aide d'un ensemble de données d'exemples étiquetés avec des annotations concernant des segments temporels dans lesquels les individus effectuaient les exercices, dans lequel une fonction de perte d'entropie binaire croisée LA est utilisée pour régler les paramètres du premier réseau neuronal (7) ;

- un deuxième réseau neuronal (8) qui, en réponse à une activité physique détectée par le premier réseau neuronal (7) et sur la base des caractéristiques extraites par le réseau neuronal dorsal (6), est configuré pour identifier l'activité physique parmi un groupe de K exercices différents pouvant être assignés à l'individu, ledit deuxième réseau neuronal (8) ayant été entraîné pour identifier les exercices à l'aide d'un ensemble de données d'exemples étiquetés avec des annotations codant l'exercice effectué par l'individu, une fonction de perte d'entropie catégorielle croisée **LE** étant utilisée pour régler les paramètres du deuxième réseau neuronal (8) afin de faire correspondre les annotations à la vérité de terrain ;

- un troisième réseau neuronal (9) qui, en réponse à l'identification d'une activité physique par le deuxième réseau neuronal (8) et sur la base des caractéristiques extraites par le réseau neuronal dorsal (6), est configuré pour compter les répétitions dudit exercice identifié, ledit troisième réseau neuronal (9) ayant été entraîné à décomposer chaque exercice en un ou plusieurs segments temporels ou phases et à appliquer une fonction de discrétisation de comptage ultérieure auxdites phases, ledit troisième réseau neuronal (9) ayant été entraîné de bout en bout avec des données annotées pour résoudre un problème de régression, où la perte Lc est une fonction de distance entre la sortie du troisième réseau neuronal et un indicateur de phase de vérification sur le terrain ;

- une version du réseau neuronal dorsal (6) qui a été adaptée aux tâches de classification binaire des exercices, de classification multiclasse des exercices et de régression de la fonction d'indicateur de phase, en affinant le réglage du réseau neuronal dorsal (6) combiné aux trois réseaux neuronaux (7), (8) et (9) sur des données étiquetées pour accomplir les tâches susmentionnées, dans lequel, au cours de l'affinement du réglage, les réseaux neuronaux (6, 7, 8, 9) ont pour instruction de minimiser une perte composée comme suit : $L = c_A * L_A + c_E * L_E + cc * L_C + c_{DA} * L_{DA}$, qui est minimisé au moyen d'une descente de gradient, où $L_{DA}$ représente la perte d'adaptation au domaine *et* $c_A$, $c_E$, $c_C$, et $C_{DA}$ sont des coefficients utilisés pour mettre à l'échelle les conditions de perte correspondantes,

dans lequel le réseau neuronal dorsal (6) est un réseau neuronal (6) résiduel convolutif comprenant une pile de convolutions paramétriques 1D, ledit réseau neuronal dorsal (6) étant configuré pour mapper une série temporelle multi-variable de dimension 18 * N dans un vecteur spatial de taille 512.

2. Système (1) selon la revendication 1, dans lequel ledit au moins un capteur inertiel (2) est un capteur inertiel à 6 axes comprenant un accéléromètre et un gyroscope.

3. Système (1) selon l'une quelconque des revendications précédentes, comprenant en outre une pluralité de canaux (3) pour transmettre des données échantillonnées à partir du capteur inertiel (2) à l'unité de normalisation (4), chacun desdits canaux (3) étant configuré pour recevoir et transmettre les données échantillonnées dans un ordre chronologique.

4. Système (1) selon l'une quelconque des revendications précédentes, dans lequel l'unité de normalisation (4) comprend un tampon pour stocker les données normalisées.

5. Système (1) selon l'une quelconque des revendications précédentes, dans lequel chacun des premier réseau neuronal (7), deuxième réseau neuronal (8) et troisième réseau neuronal (9) est un perceptron multicouche à 2 couches.

6. Système (1) selon l'une quelconque des revendications 1 à 4, dans lequel le premier réseau neuronal (7), le deuxième réseau neuronal (8) et/ou le troisième réseau neuronal (8) est choisi parmi une combinaison de l'une des architectures suivantes : couches convolutives, couches récurrentes, MLCT, couches d'attention, couches entièrement connectées, forêt d'arbres de décision aléatoires, forêt d'arbres de décision boostés par le gradient, machine à vecteurs de support, regroupement par la méthode des K centroïdes, régression polynomiale, régression logistique.

7. Système (1) selon l'une quelconque des revendications précédentes, dans lequel chaque phase d'un exercice est modélisée par une fonction d'indicateur de phase f(t) ayant les propriétés suivantes :

• f(t) est comprise dans l'intervalle [0, 1] ;
• f(t) = 0 si l'individu n'effectue pas l'exercice ;
• avec Tb désignant le début de la phase d'exercice et Te désignant la fin de la phase d'exercice, f(Tb) = f(Te) = 0 ;

• avec Tm désignant le point médian de la phase d'exercice, où Tb < Tm < Te, f(Tm) = 1 ;
• f(t) augmente de façon monotone dans [Tb, Tm[ ;
• f(t) diminue de façon monotone dans [Tm, Te[.

8. Système (1) selon la revendication 7, dans lequel la fonction d'indicateur de phase f(t) est mise en œuvre comme une fonction en dents de scie lissée, qui est lissée par un noyau de Gauss.

9. Système (1) selon la revendication 8, dans lequel le troisième réseau neuronal (9) inclut une machine à états finis, FSM, configurée pour accepter une prédiction de régression lissée de ladite fonction d'indicateur de phase f(t) et pour sortir soit la valeur numérique « 1 » lorsqu'une phase de répétition d'un exercice a été achevée, soit la valeur numérique « 0 » dans tous les autres cas, sur la base du moment où ladite prédiction de régression lissée satisfait à certaines conditions logiques prédéfinies qui sont une combinaison de l'un des éléments suivants : seuils sur la valeur de la fonction, seuils sur le gradient, seuils sur les dérivées de second ordre ou d'ordre supérieur, seuils adaptatifs basés sur l'état et/ou les sorties de la FSM.

10. Procédé (100) mis en œuvre par ordinateur d'identification, d'analyse et de comptage de répétitions d'exercices physiques par un individu utilisant le système (1) selon l'une quelconque des revendications précédentes, le procédé (100) comprenant les étapes suivantes :

- disposer au moins un capteur inertiel sur une partie du corps de l'individu (101) ;
- échantillonner des signaux étant représentatifs de paramètres biométriques et/ou médicaux de l'individu à l'aide dudit au moins un capteur inertiel afin d'obtenir des données échantillonnées (102) ;
- normaliser les données échantillonnées (103) ;
- concaténer les données échantillonnées normalisées afin d'obtenir un flux unique de données sous la forme d'une série temporelle multi-variable (104) ;
- extraire des caractéristiques de la série temporelle multi-variable à l'aide du réseau neuronal dorsal dudit système, ledit réseau neuronal dorsal ayant été pré-entraîné par une technique (105) de cadre simple pour l'apprentissage contrastif de la représentation visuelle, SimCLR ;
- détecter des activités physiques en réponse à la réception des caractéristiques extraites à l'aide du premier réseau neuronal dudit système (106) ;
- en réponse à une activité physique détectée par le premier réseau neuronal et sur la base des caractéristiques extraites par le réseau neuronal dorsal, identifier l'activité physique parmi un ensemble de K exercices différents au moyen du deuxième réseau neuronal dudit système (107) ;
- en réponse à l'identification d'une activité physique par le deuxième réseau neuronal et sur la base des caractéristiques extraites par le réseau neuronal dorsal, compter les répétitions dudit exercice identifié au moyen du troisième réseau neuronal dudit système, ledit troisième réseau neuronal ayant été entraîné à décomposer chaque exercice en un ou plusieurs segments temporels ou phases (108).

11. Procédé (100) selon la revendication 10, dans lequel chaque phase d'un exercice est modélisée par une fonction d'indicateur de phase f(t) ayant les propriétés suivantes :

• f(t) est comprise dans l'intervalle [0, 1] ;
• f(t) = 0 si l'individu n'effectue pas l'exercice ;
• avec Tb désignant le début de la phase d'exercice et Te désignant la fin de la phase d'exercice, f(Tb) = f(Te) = 0 ;
• avec Tm désignant le point médian de la phase d'exercice, où Tb < Tm < Te, f(Tm) = 1 ;
• f(t) augmente de façon monotone dans [Tb, Tm[ ;
• f(t) diminue de façon monotone dans [Tm, Te[.

12. Procédé (100) selon la revendication 11, dans lequel l'étape de comptage des répétitions (108) comprend les sous-étapes suivantes :

- appliquer un filtre passe-bas lisseur pour éliminer les oscillations parasites et réduire la quantité de bruit dans la fonction d'indicateur de phase ;
- initialiser un compteur de phase à N, c'est-à-dire le nombre de phases composant l'exercice ;
- jusqu'à ce que le compteur de phase soit à zéro, répéter un cycle comprenant :

• initialiser une machine à états finis du troisième réseau neuronal à un état initial ;
• dès que la fonction d'indicateur de phase filtrée satisfait certaines conditions logiques, appliquer une transition d'état à l'état qui est fixé à un exercice d'état, sinon il est laissé inchangé ;
• dès que la fonction d'indicateur de phase filtrée satisfait d'autres conditions logiques, réinitialiser l'état à l'état initial et diminuer le compteur de phase d'une unité ;

- lorsque le compteur de phase atteint zéro, augmenter le nombre de répétitions détectées d'une unité et réinitialiser le compteur de phase à N.

13. Programme informatique, ayant des instructions qui, lorsqu'elles sont exécutées par un dispositif informatique, amènent le dispositif informatique à mettre en œuvre le procédé selon l'une quelconque des revendications 10 à 12.

FIG. 1a

FIG. 1b

FIG. 2

## FIG. 3

FIG. 4

## FIG. 5

## FIG. 6

EP 4 407 628 B1

# FIG. 7

| Difficulty Level | Definition |
|---|---|
| 1 | • Simple basic movements **(static or mono-lateral)**<br>• from a comfortable initial position<br>• involving **one single limb** and mostly **1 joint** |
| 2 | • **BS,** targeting mainly one joint and a single plane<br>• static or mono-lateral exercise starting from an **uncomfortable position** (lying down) or requiring **balance** or **BS** movements<br>• BA balance monopodalic exercises with support or bipodalic exercises without support<br>• no effort required |
| 3 | • **BA** with **one target joint and/or single main movement plane**<br>• **BS** from an **uncomfortable** starting position or composed of movement on **multiple planes** or **small amplitude movements** with **equipment** (e.g. elastic bands) are also included |
| 4 | • **BA** exercises targeting **multiple planes and multiple joints**<br>• BS or BA with movement on multiple planes **starting to making effort** (e.g. using **elastic bands** or weights) and/or with **higher duration** of the single repetition (i.e. **sequence** of different movements on multiple planes or **large** movements) or **requiring coordination of multiple limbs** |
| 5 | • **requiring effort**<br>• requiring **coordination of multiple limbs** without or with **objects**<br>• BA exercises targeting multiple planes and multiple joints starting from an **uncomfortable** position **(lying down)** |

FIG. 8

## FIG. 9

100

**101** – ARRANGING AT LEAST ONE INERTIAL SENSOR IN A BODY PART OF THE INDIVIDUAL

**102** – SAMPLING SIGNALS USING THE AT LEAST ONE INERTIAL SENSOR SO AS TO OBTAIN SAMPLED DATA

**103** – NORMALIZING THE SAMPLED DATA

**104** – CONCATENATING THE NORMALIZED SAMPLED DATA SO AS TO OBTAIN A SINGLE FLOW OF DATA IN THE FORM OF A MULTIVARIATE TIME SERIES

**105** – EXTRACTING FEATURES FROM THE MULTIVARIATE TIME SERIES BY USING A BACKBONE NEURAL NETWORK THAT HAS BEEN PRE-TRAINED BY SimCLR TECHNIQUE

**106** – DETECTING EXERCISE EVENTS IN RESPONSE TO THE RECEIPT OF THE EXTRACTED FEATURES BY USING A FIRST NEURAL NETWORK

**107** – IN RESPONSE TO AN EXERCISE EVENT DETECTED BY THE FIRST NEURAL NETWORK AND, BASED ON THE FEATURES EXTRACTED BY THE BACKBONE NETWORK, IDENTIFYING THE EXERCISE EVENT OUT OF A POOL OF K DIFFERENT EXERCISES BY MEANS OF A SECOND NEURAL NETWORK

**108** – IN RESPONSE TO THE IDENTIFICATION OF AN EXERCISE EVENT BY THE SECOND NEURAL NETWORK AND BASED ON THE FEATURES EXTRACTED BY THE BACKBONE NEURAL NETWORK, COUNTING REPETITIONS OF THE IDENTIFIED EXERCISE BY MEANS OF A THIRD NEURAL NETWORK THAT HAS BEEN TRAINED TO DECOMPOSE EACH EXERCISE IN ONE OR MORE PHASES

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3627514 A1
- AU 2020101986 A4
- WO 2020205276 A1
- US 2022080262 A1

**Non-patent literature cited in the description**

- **TING CHEN et al.** Simple Framework for Contrastive Learning of Visual Representations. *ICML'20 Proceedings of the 37th International Conference on Machine Learning*, July 2020 (149), 1597-1607
- **SHAI BEN-DAVID et al.** *A theory of learning from different domains*, https://link.springer.com/content/pdf/10.1007/s10994-009-5152-4.pdf